# EUROPEAN PATENT APPLICATION

(11) **EP 0 577 178 A1**
(43) Date of publication of application: **05.01.1994**
(21) Application number: 93201762.7
(22) Date of filing: 18.06.1993
(51) Int. Cl.: A61F 2/44

(54) **An implant having an open-cell or open-pore metal structure**

(30) Priority: 20.06.1992 DE 4220218
(71) Applicant: ESKA Implants GmbH & Co., 23556 Lübeck (DE)
(72) Inventor: Schug, Martin, D-23552 Lubeck (DE)
(74) Representative: Fuchs, Luderschmidt & Partner Patentanwälte

(57) **Abstract**

An implant (1) having an open-cell or open-pore metal structure for fixing adjacent vertebrae is provided. The implant contains several implant disks (2) which can be laid one on top of the other, a bore (6) through the implant disks (2) and a connecting means (30) passing through the bore (6), which connects the implant disks to form a secure disk unit.

## Description

The invention relates to an implant having an open-cell or open-pore metal structure for fixing adjacent vertebrae, which are cut away on opposing surfaces of the body of the vertebra.

Such implants are known for example from EP-A-O 271 501, which after the partial removal of adjacent vertebrae are inserted into the created recess so that the adjacent osseous tissue can grow into the open-cell or open-pore metal structure of the implant, as a result of which the desired rigid connection of the two adjacent vertebrae is achieved. At the opposing bone surfaces, which abut against the adjacent vertebrae and are subject to the pressure loading of the vertebral column, the implant possesses ring faces made from solid metal, which form working surfaces of the tool and are to facilitate the insertion of the implant into the recess. However the provision of these solid ring faces reduces the active face, provided for ingrowth of the abutting osseous tissue in the external region. Furthermore, in the case of the known implant, because of its predetermined length it is only possible to adapt to the patient's individual requirements by holding a large stock of varying sizes.

The object of the invention is to develop the implant of the aforementioned type further so that an adaption of the size to the individual requirements of the patient is simplified and the connection between the vertebral bodies and the implant is improved.

This object is achieved with the implant of the aforementioned type in accordance with the invention by an implant comprising several discs which can be laid one on top of the other, through which connecting means for connecting the disks pass.

The advantages of the invention lie in particular in that the implant is made from several implant disks of which a desired number are disposed on top of one another and are securely connected to one another so that - during the operation - according to the findings relating to the disease, an implant, which has the desired axial length corresponding to the number of implant disks, is constructed. A countersunk screw, for example, which passes through the substantially central bore, is used as connecting means, and the end faces of the implant opposite one another are constructed with open cells or open pores in their radial external region, so that the implant can also fuse with the adjacent vertebral bodies on the front side as well.

In a particularly preferred manner the implant disks have plane end faces, which may extend either perpendicular or slightly inclined to the axis of the bore, whereby e.g. the outer end faces of the two outer implant disks may be slightly inclined in opposite directions, in order to confer the disk unit with an overall conical shape, which was previously prepared in a corresponding wedge shape. The inner implant disks inserted between the outer implant disks are then preferably provided with two plane and parallel end faces, so that should a wedge angle of the total implant be desired it is only produced by the outer end faces of the outer implant disks.

In a particularly preferred manner the implant disks are constructed so that they are rotationally symmetrical about their bore axis, they preferably have the shape of a cylinder or cone, and the end faces of adjacent disks correspond to have an identical shape in order to prevent gaps in the diameter or local steps in the disk unit.

According to a preferred embodiment of the invention, one of the outer disks is provided with a countersunk bore to house a screw head. The other outer implant disk possesses an internal thread in its bore, and a countersunk screw, which passes through all the bores, lying with its head in the countersunk bore of one outer implant disk and with its tail in the other disk, to act as the connecting means. The desired number of intermediate disks, the bores of which are preferably not threaded, are inserted between the two outer implant disks. As soon as the screw engages in the internal thread of the corresponding outer implant disk and is tightened a secure disk unit is produced whereby the disk may if necessary also be locked by a lock nut (not shown).

In a particularly preferred manner, axial pegs and/or corresponding recesses are disposed on the implant disks, in order to fix adjacent disks in a corresponding angular position in which the peg engages in the corresponding recess so that adjacent disks abut one another without being able to rotate.

The implant disks advantageously have a central core made from solid metal, to which the open-pore or open-cell metal structure is radially connected. The bore through the implant disks extends into the central core so as to enable a secure attachment between connection means and the implant disks.

Advantageous refinements of the invention are characterised by the features of the subordinate claims.

An exemplified embodiment of the invention is explained in further detail below by reference to the drawings.
- Fig. 1: shows a longitudinal section through the components of an implant before the connection of the implant disks;
- Fig. 2: shows a longitudinal section corresponding to Fig. 1 through the implant after the connection of the implant disks;
- Fig. 3: shows the position of the implant between two adjacent vertebrae to be connected rigidly to one another, and
- Fig. 4: shows a partially inner view of the vertebral column with a fastening implant

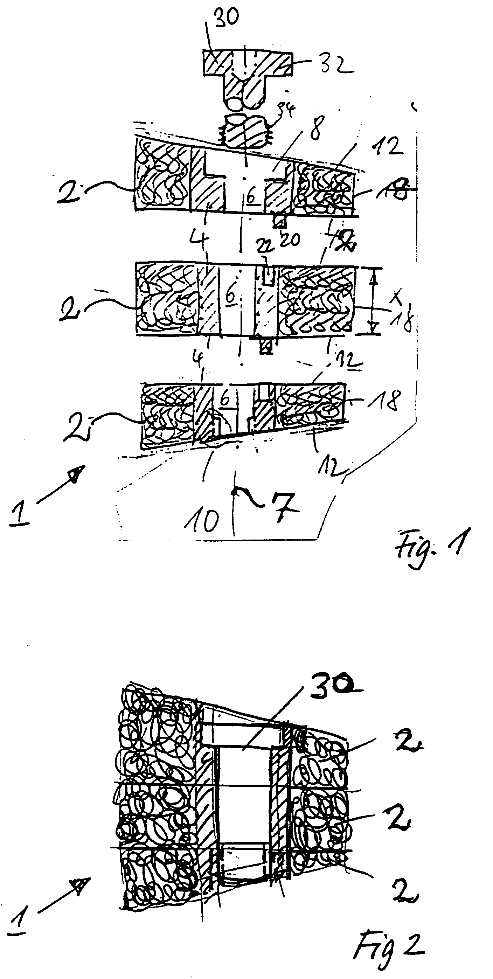

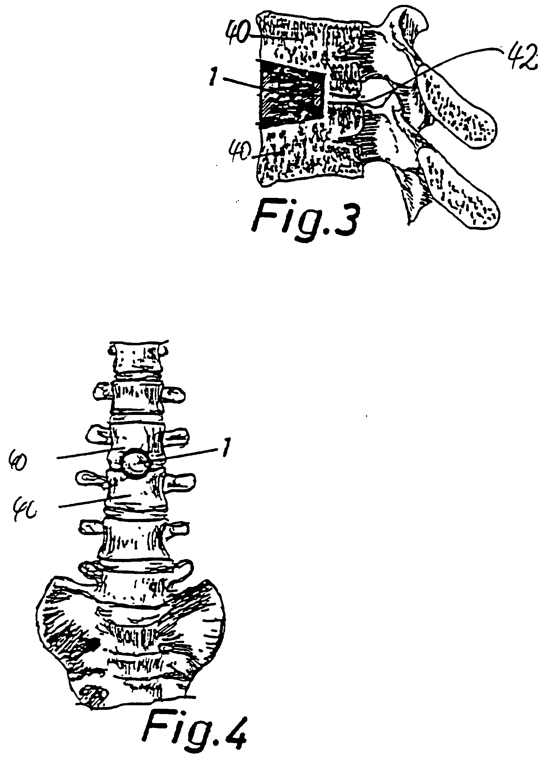

Fig. 1 and 2 respectively show in an exploded representation and in a ready assembled form an implant 1, which is used for fixing adjacent vertebrae. Fig. 3 and 4 show the implant 1 in the implanted state between two adjacent vertebrae 40, the opposing bone surfaces 42 of which are cut away so that a recess 44 for housing the implant 1 is produced.

In the embodiment represented in Fig. 1 and 2, the implant 1 contains several implant disks 2 disposed one above the other, which have a cylindrical shape. Through the centre of the implant disks 2 extends a bore 6, and through the bore 6 extends a screw 30, which holds the implant disks 2 together to form a disk unit.

The disks 2 all have a central core 4 made from solid metal, through which the bore 6 passes. The solid core 4 passes radially at a predetermined distance from the axis 7 of the bore 6 into an open-cell or open-pore metal structure 16, outer disk 2 and the disk unit is connected in this way to form an implant 1.

The implant 1 may just be constructed from the two outer implant disks 2, but as an alternative it may also have several intermediate disks having the same or different height between the two outer implant disks 2.

The implant 1 is then inserted into a space 44, which as shown in Fig. 3 and 4 is prepared between adjacent vertebral bodies 40 in the vertebral column. Because of the open-cell or open-pore metal structure, over a large area the adjacent osseous tissue grows into the open cells or pores of the implant and produces the required rigidity of the vertebral bodies.

## Claims

1. An implant having an open-cell or open-pore metal structure for fixing adjacent vertebrae **characterized in that** the implant comprises several implant disks (2) that can be laid one on top of another, a bore (6) passing through the implant disks (2), and connecting means (30) passing through the bore (6) for connecting the implant disks (2).

2. An implant according to Claim 1, **characterized in that** the implant disks (2) possess planar front faces (12).

3. An implant according to Claim 1 or 2, **characterized in that** the front faces (12) of the implant disks (2) extend at an incline to the axis of the bore (6).

4. An implant according to one of the preceding Claims, **characterized in that** the implant disks (2) have a cylindrical or conical shape.

5. An implant according to one of the preceding Claims, **characterized in that** the connecting means (30) comprise a screw, a countersunk bore (8) in one of the outer disks (2) to receive the screw head (32), and an internal thread (10) in the bore (6) of the other outer disk (2) to receive the thread (34) of the screw (30).

6. An implant according to one of the preceding Claims, **characterized in that** on their front faces (12) the implant disks (2) have axial pegs (20) and/or corresponding recesses (22) which engage in one another to prevent rotation.

7. An implant according to one of the preceding Claims, **characterized in that** the disks (2) have a central core (4) made from solid metal, through which the bore (6) passes, and at its periphery has an open-pore or open-cell metal structure (16).
